# EUROPÄISCHE PATENTSCHRIFT

(11) **EP 1 652 536 B1**
(45) Veröffentlichungstag und Bekanntmachung des Hinweises auf die Patenterteilung: **09.12.2009**
(21) Anmeldenummer: 04024140.8
(22) Anmeldetag: 09.10.2004
(51) Int. Cl.: A61K 47/48, A61K 33/06, A61K 33/12, A61K 33/18, A61P 31/02, A61P 7/04, A61L 15/18, A61L 15/46

(54) **Zeolith-Jod-Einschlussverbindung zur Behandlung von Wunden**
Zeolite iod clathrate for the treatment of wounds
Composé d'inclusion de zéolite-iode pour le traitement des plaies

(43) Veröffentlichungstag der Anmeldung: 03.05.2006
(73) Patentinhaber: Koman, Karl, 5026 Salzburg (AT)
(72) Erfinder: Koman, Karl, 5026 Salzburg (AT); Arnold, Claudia, Dr., 88481 Balzheim (DE); Rüegger, Urs, 4800 Zofingen (CH)
(74) Vertreter: von Kreisler Selting Werner

(56) Entgegenhaltungen:
- WO-A-02/22060
- US-A1- 2002 066 702
- SOPKOVA A ET AL: "Zeolites and veterinary pharmacy" S.T.P. PHARMA SCIENCES 1994 FRANCE, Bd. 4, Nr. 5, 1994, Seiten 366-372, XP009045037 ISSN: 1157-1489
- DATABASE WPI Section Ch, Week 199809 Derwent Publications Ltd., London, GB; Class B06, AN 1998-099209 XP002320928 & RU 2 083 224 C1 (PODCHAINOV S F) 10. Juli 1997 (1997-07-10)

## Beschreibung

Die Erfindung betrifft eine Zeolith-Iod-Einschlussverbindung, die Verwendung der Zeolith-Iod-Einschlussverbindung sowie definierte Materialien zur Behandlung von Wunden enthaltend die Zeolith-Iod-Einschlussverbindung.

Elementares Iod wird seit langer Zeit zur Desinfektion von Wunden verwendet. Es ist ein wirksames Mikrobizid, welches keine Resistenzen hervorruft und durch die Desinfektion der Wunde eine schnelle Wundheilung ohne Entzündung oder lokale bzw. systemische Infektion fördert. Elementares Iod als solches kann nicht eingesetzt werden; es bedarf einer Zubereitung, beispielsweise der alkoholischen Iodtinktur.

Iod wird bevorzugt in Verbindung mit einem Iodophor verwendet. Ein Iodophor ist ein Träger von Iod, welcher den Wirkstoff langsam freisetzt und so seine Wirkung verlängert, außerdem die lokale Konzentration gering hält und so die Wirksamkeit verbessert. Ein typischer Iodophor ist Polyvinyl-Pyrrolidon. Um diese Zubereitungen anzuwenden, ist es jedoch notwendig, dass zuerst der Blutfluss gestoppt wird, damit das Iod auf die Wundfläche gelangen kann. Zur Blutstillung bei großflächigen oder tiefen Wunden werden eine ganze Reihe von Verbindungen gebraucht, deren Wirkung meistens darin besteht, Eiweißstoffe aus dem Blut auszufällen und so die Bildung von Schorf zu beschleunigen, oder aber Feuchtigkeit zu binden und mit dem Blut zu einem Wundverschluss zu verkleben. Diese Mittel können traditioneller Natur sein wie Kaffeemehl, oder sie können Metallsalze wie Aluminiumhydroxid oder auch biochemische Wirkstoffe enthalten, welche direkt in den Vorgang der Blutkoagulation eingreifen. Ein bekanntes Mittel ist Zeolith, welcher sich bei der Blutstillung durch einen besonders schnellen Wirkungseintritt auszeichnet. EP 0 888 783B1 beschreibt die Verwendung von Zeolithen zur Blutstillung und Förderung der Wundheilung.

Zeolithe zeichnen sich dadurch aus, dass sie in Bezug auf ihre Masse bis zu 25 % Wasser adsorbieren können. Auf diese Weise können sie für eine Konzentration der Gerinnungsfaktoren und für eine schnelle Gerinnung des Blutes sorgen.

US 4,822,349 beschreibt ein solches Verfahren zur Blutungsstillung von warmblütigen Säugetieren einschließlich des Menschen, bei der steriler, dehydrierter Zeolith unmittelbar oder mittels eines Verbandes auf die Wunde aufgebracht wird, so dass das Molekularsieb durch Konzentration den Koagulationseffekt bewirkt. RU 2143908 C1 beschreibt die Anwendung von zeolithhaltigem Tuff, RU 2185149 C1 die Verwendung von flüssigen oder pulverförmigen zeolithhaltigen Zubereitungen zur Behandlung von Hautverletzungen.

Eine Besonderheit des Zeoliths ist, dass er nicht unmittelbar mit dem Blut gemischt werden muss, um seine Wirkung zu entfalten. WO 02/30479 beschreibt die Verwendung von Zeolith in Vliespäckchen zur Gerinnungsbeschleunigung. Die Feuchtigkeit wird auch dann absorbiert, wenn der Zeolith räumlich nicht zu weit von der Flüssigkeitsansammlung getrennt ist, so beispielsweise durch ein Vlies, das zwischen dem Zeolith und der Wunde liegt. Ein besonderer Vorteil liegt darin, dass die Wunde so nicht durch den mineralischen Fremdstoff verkrustet wird, sondern sauber bleibt.

WO 02/22060 A1 beschreibt Kompressen zur Wundbehandlung, die zeolithisches Füllmaterial enthalten, wobei dieses zeolithische Füllmaterial permanent mit antimikrobiellem Silber beladen ist. Hierdurch werden zwar Blutkoagulation und desinfizierende Wirkung erreicht, jedoch ist die wasseraufnehmende Wirkung und damit die Gerinnungsbescheunigung wegen der permanenten Silberbeladung des Zeolith verbesserungsfähig, und für eine desinfizierende Wirkung ist ein tatsächlicher oder nahezu direkter Kontakt mit der Wunde notwendig, der die Gefahr der Beschädigung gesunder Zellen in sich trägt.

Auch US 2001/0009831 A1 beschreibt die Verwendung von permanent Silber-beladenen Zeolithen als Bestandteil von antimikrobischen Wundabdeckungen.

EP 0 837 077 B1 beschreibt antibakterielle wasser- und geruchsabsorbierende Zusammensetzungen für Einwegwindeln, Hygienetücher, Stilleinlagen und medizinische Einwegunterlagen, die wenigstens ein aus Partikeln bestehendes wasserabsorbierendes Harz und wenigstens ein antibakterielles Pulver enthalten, wobei das antibakterielle Pulver wenigstens ein aus Partikeln bestehendes anorganisches Adsorptionsmittel enthält, in das wenigstens ein antibakterielles Mittel adsorbiert ist, wobei es sich um C₆₋₈-aliphatische Alkyl-quaternäre Ammoniumsalzverbindungen, Benzalkoniumsalzverbindungen, Chlorhexidinverbindungen und/oder Polymethylenbiguanidinverbindungen handelt.

A. Sopová et al., Zeolites and veterinary pharmacy, S. T. P. Pharma Sciences 1994, France, Bd. 4, Nr. 5, 1994, S. 366 - 372, ISSN: 1157-1484 beschreibt die desinfizierenden Eigenschaften einer Zeolith-Iod-Einschlussverbindung.

Weiterhin besteht daher Bedarf an einem universell einsetzbaren Wundbehandlungsmittel, das zum einen die Blutgerinnung fördert und zum anderen effektiv, nebenwirkungsarm und unmittelbar an und in der Wunde langanhaltend desinfizierend wirkt.

Die Aufgabe wird erfindungsgemäß gelöst durch eine Zeolith-Iod-Einschlussverbindung zur Anwendung bei der Behandlung von Wunden bei Menschen und Tieren.

Der Begriff Zeolith im Sinne der vorliegenden Erfindung steht für eine weitverbreitete Gruppe von kristallinen Silicaten, und zwar grundsätzlich von wasserhaltigen Alkali- und/oder Erdalkali-Alumosilicaten der allgemeinen Formel

M_{2/z}O Al₂O₃ xSiO₂ yH₂O

wobei M für ein ein- oder mehrwertiges Metall, insbesondere Alkali- oder Erdalkali-Kation, H oder NH₄, z für die Wertigkeit des Kations, x für eine Zahl im Bereich von 1,8 bis 12 und y für eine Zahl im Bereich von 0 bis etwa 8 steht. Das stöchiometrische Verhältnis von SiO₂ zu Al₂O₃ (Modul) ist eine wichtige Kennzeichnungsgröße der Zeolithe. Charakteristisch für die meisten Zeolithen ist, dass sie ihr Wasser beim Erhitzen stetig und ohne Änderung der Kristallstrukturen abgeben, andere Verbindungen anstelle des entfernten Wassers aufnehmen und so durch die Austauschreaktion Einschlussverbindungen, beispielsweise mit Iod eingehen. Erfindungsgemäß eingesetzte Zeolithe enthalten zur Bildung der Iod-Einschlussverbindung kein oder nur geringe Menge an Wasser.

Dadurch, dass Zeolith ein starkes Adsorptionsmittel ist, wird er befähigt, viel Wasser zu binden, er kann aber auch andere Stoffe in seinen Nanoporen tragen, beispielsweise Iod. Da die Bindung an Wasser jedoch stärker ist als die an Iod, wird, sobald der mit Iod beladene Zeolith mit Wasser, beispielsweise aus Blut, in Berührung kommt, das Iod in feinverteilter Form freigesetzt und das Wasser gebunden. Hiermit wird ein sofortiger Blutstillstand bewirkt.

Dadurch wird erfindungsgemäß ein synergistischer Effekt zwischen Gerinnungsbeschleunigung durch Wasseraufnahme in dem Zeolith und Desinfektion durch gleichzeitige kontrollierte Abgabe des Iods erzielt, da der iodbeladene Zeolith die Eigenschaften eines Iodophors mit denen einer blutstillenden Substanz vereint. Bei der Anwendung wird so in einem Schritt die Wunde getrocknet und desinfiziert. Der Zeolith absorbiert das Wasser aus dem Blut, nicht aber die anderen Blutbestandteile (abgesehen von den Salzen). Wasser verdrängt das auf dem Zeolith adsorbierte Iod, dieses tritt aus. Durch die Entfernung von Wasser werden die Gerinnungsfaktoren so weit angereichert, dass die Koagulation deutlich beschleunigt eintritt.

Die erfindungsgemäße Zeolith-Iod-Einschlussverbindung (Komplex) lässt sich prinzipiell für die Wundbehandlung und Behandlung von Abschürfungen und tiefen Wunden, wie Schnittverletzungen, an allen Lebewesen einsetzen, deren Blut spontan gerinnt, insbesondere an Menschen und an allen warmblütigen Tieren.

Der Komplex kann prinzipiell aus einem beliebigen Zeolith bestehen; beste Ergebnisse werden jedoch mit Ca- oder Na/Ca-Zeolithen erzielt. Der Zeolith der Zeolith-Iod-Einschlussverbindung weist vorzugsweise eine Porengröße von mindestens 5 Å **(0,5 nm)** auf. Bei kleineren Porengrößen wird keine stabile Zeolith-Iod-Einschlussverbindung gebildet, da das Iod in den Poren des Zeoliths keinen Platz findet. Ein solches Produkt trägt nur wenig oberflächlich adsorbiertes Iod in sich.

Besonders bevorzugt ist, wenn der Zeolith der Zeolith-Iod-Einschlussverbindung mit Iod im Bereich von 0, 1 Gew.-% bis zu seiner Maximalkapazität, insbesondere im Bereich von 1 bis 10 Gew.-% beladen ist, um eine effiziente Desinfizierung zu erzielen. Dabei ist das Iod bevorzugt im Wesentlichen in den Nanoporen des Zeoliths der Zeolith-Iod-Einschlussverbindung physikalisch gebunden.

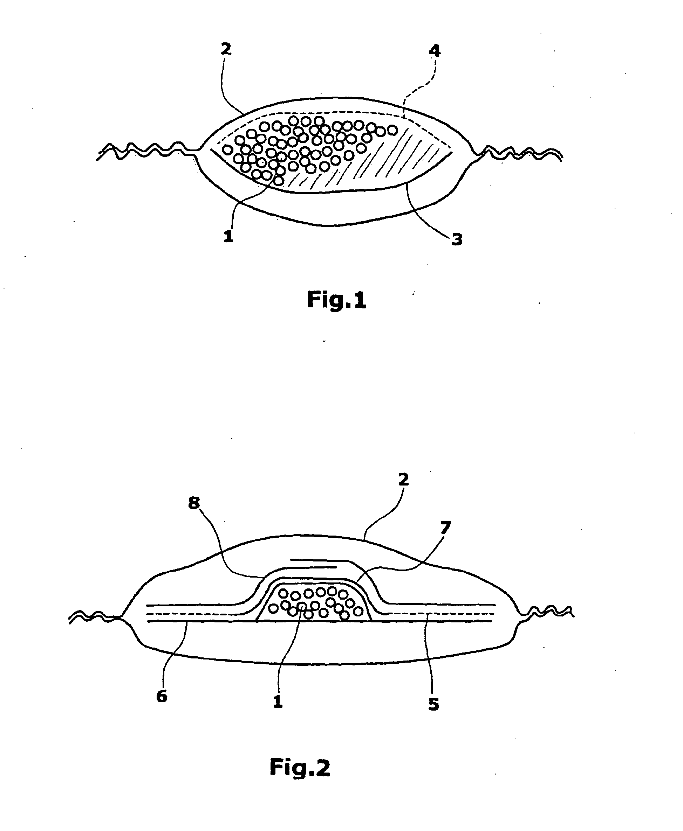

Der Zeolith-Iod-Komplex kann granuliert, pelletiert, gekörnt oder pulverförmig sein, wobei beste Koagulationsergebnisse bei einer mittleren Korngröße der Zeolith-Iod-Einschlussverbindung von 0,5 bis 2 mm, insbesondere 1 mm erzielt werden. Die Zeolith-Iod-Einschlussverbindung kann in einem losen Kissen oder Beutel mit einer porösen Seite angewendet werden, wie in Fig. 1 dargestellt ist. Diese wird auf die Wunde gedrückt. Der Beutel kann abgenommen werden, wenn die Wunde trocken oder die gefühlte Hitze zu groß ist. Eine typische Menge sind 1 bis 2 g Zeolith-Iod-Einschlussverbindung in einem losen Beutel, einsprechend einer Einzeldosis Iod von max. 50 mg bei der gemessenen Freisetzung.

Wundbehandlungsmittel können neben den essentiellen Bestandteilen Zeolith und Iod auch weitere kompatible Hilfs- und Zusatzstoffe, beispielsweise weitere Wundheilungsmittel oder neutrale Füllstoffe enthalten.

Zur schnellen Versorgung großflächiger Wunden können ein oder mehrere solcher Beutel auch aufgerissen werden. In Fig. 1 wird ein entsprechender Beutel in ein einem Folienbeutel dargestellt.

Das Präparat kann auch in kleinen Mengen (250 mg) in ein selbstklebendes Wundpflaster integriert werden. Aufgrund der Hitzeentwicklung sollten dünne Schichten oder lose Pads eingesetzt werden, die man schnell abnehmen und wieder auflegen kann. In Fig. 2 wird ein entsprechendes Pflaster im Folienbeutel dargestellt.

Wichtig für die Anwendung ist, dass das Produkt vor der Verwendung trocken aufbewahrt wurde. Es ist in der Praxis unerlässlich, dass jede Portion einzeln in einer wasserdichten und ioddichten Umverpackung aufbewahrt wird. Eine gute Umverpackung besteht beispielsweise aus relativ zäher, dicker Metallfolie oder metallbeschichteter Folie, wie sie beispielsweise für Suppositorien eingesetzt wird.

Typische Situationen, in denen ein Wundbehandlungsmittel Anwendung findet, sind solche, in denen ein gewöhnliches Pflaster ungünstig oder unerwünscht wäre, etwa bei Personen oder bei Tieren, die auf den Klebstoff allergisch sind etc. Außerdem ist es günstig, wenn die Blutung sehr schnell gestoppt werden muss, oder wenn die Wunde infiziert sein könnte, etwa bei Kratzern durch Tiere oder bei Stürzen im Sport, beispielsweise Fußball oder Radsport.

Insbesondere bevorzugt ist, dass die Zeolith-Iod-Einschlussverbindung das Iod im Medium Ethanol/Wasser (1:8 v/v) bei Raumtemperatur innerhalb von 20 min. etwa 16 % der theoretisch zu erwartenden Menge Iod freisetzt.

Die erfindungsgemäßen Wundbehandlungsmittel können je nach Verwendungszweck zusätzlich beispielsweise synthetische und/oder natürliche Polymere, beispielsweise Polyacrylate, SIBS-Verbindungen, SEBS-Verbindungen, Naturkautschuke, Chitosane, Alginate, Hydrogele, Hydrokolloide und/oder Polyurethane enthalten.

Zur Verstärkung der desinfizierenden Wirkung und/oder je nach Anwendungszweck können sie des weiteren insbesondere oxidierende anorganische und/oder organische Verbindungen, desinfizierende chemische Verbindungen, übliche Füll- und Hilfsstoffe, Duftstoffe und/oder Farbstoffe enthalten.

Erfindungsgemäß finden die beschriebenen Zeolith-Iod-Einschlussverbindungen Verwendung zur Herstellung von wundstillenden Materialien, insbesondere Kompressen, Pflaster und/oder Verbandmaterial.

Wundbehandlungsmittel im Sinne der Erfindung können die Zeolith-Iod-Einschlussverbindung in Form einer Wundkompresse mit einer porösen Fläche enthalten, welche auf die Wunde gelegt wird. Die Kompresse kann entweder Bestandteil eines Pflasters oder lose sein. Ebenso kann das Wundbehandlungsmittel auf andere Weise auf das Pflaster aufgebracht oder darin enthalten sein oder Bestandteil eines Verbandmaterials sein.

Bei großflächigen Verletzungen ist es auch möglich, die erfindungsgemäße Zeolith-Iod-Einschlussverbindung direkt auf die Wundfläche zu streuen.

Diese Behandlung kann am Menschen oder am Tier erfolgen.

Darüber hinaus stellen Einwegwindeln, Damenbinden oder Stilleinlagen bevorzugte Ausführungsformen der Erfindung dar. Wundstillende Materialien im Sinne der Erfindung können ferner beispielsweise Pasten oder poröse oder nichtporöse Materialien wie Filme, beispielsweise Siloxanfilme, Polyesterfilme, Polyurethanfilme sein. Hier ist jedoch zu beachten, dass die Grundlage der Paste keine Materialien enthält, die von dem Zeolith adsorbiert werden.

Das Material zur Anwendung bei der Behandlung von Wunden ist bevorzugt dadurch gekennzeichnet, dass das Wundbehandlungsmittel wundseitig von einem porösen textilen Faserstoff, insbesondere einem Vlies, Gewirk oder Gestrick sowie mit einer feuchtigkeitsdurchlässigen Folie, abgedeckt ist, welcher mit 0,1 % bis zu seiner Maximalkapazität, vorzugsweise mit 1 bis 10 % Iod beladen ist, dergestalt, dass das Iod sich in den Nanoporen des Zeolithen gebunden befindet. Ausführungsbeispiele

### a) Herstellung der Zeolith-Iod-Einschlussverbindung

In einem Exsikkator wurde 150 g wasserfreier Zeolith 5 Å **(0,5 nm)** (Aldrich) pelletiert eingewogen. In einer separaten Kristallisierschale wurden 15 g (10 %) Iod eingewogen. Die Schale wurde in das Molekularsieb gestellt, ohne dieses mit dem Iod in direkten Kontakt zu bringen. Der verschlossene Exsikkator wurde evakuiert und auf 50 °C temperiert. Der vollständige Übergang des Iods auf das Molekularsieb erfolgte innerhalb von 2 Tagen.

Dieselbe Versuchsanordnung wurde auch mit 1 % Iod ausgeführt, der Versuch dauerte in diesem Fall nur 2 Stunden. Bei der alternativen Durchführung mit einem Überschuss an Iod musste das Molekularsieb noch einige Stunden für sich ins Vakuum gestellt werden, um es von oberflächlich anhaftendem Iod zu befreien.

### b) Iodfreisetzung

5 g Zeolith-Iod-Komplex (10 % Iod) wurden mit 10 ml Ethanol und 80 ml demineralisierten Wasser versetzt und 20 min lang gerührt. Anschließend wurde das Iod nach dem üblichen Verfahren mit Natrium-Thiosulfat titriert. Die Iodfreisetzung erfolgte langsam und zeitabhängig. Nach 20 min waren 16 % der theoretisch zu erwartenden Menge freigesetzt.

### c) Mikrobiologische Wirksamkeit

45 ml physiologische Kochsalzlösung wurden mit einer Keimsuspension von Staph. aureus versetzt, so dass eine Konzentration von 1,5 · 10⁷ KBE/ml vorlag. Zu dieser Keimsuspension wurde 1 g Zeolith-Iod-Komplex (10 %) gegeben und leicht geschüttelt. Nach verschiedenen Zeitabständen wurde je 1 ml entnommen und die Zahl der überlebenden Keime mittels Plattengussverfahren in TSA-Agar bestimmt. Die Inkubation erfolgte bei 37 °C für 24 h.
Probennahme nach 1 min: starkes Wachstum
Probennahme nach 5 min: kein Wachstum
Probennahme nach 15 min: kein Wachstum
Probennahme nach 30 min: kein Wachstum.

Demnach kann festgestellt werden, dass der Zeolith-Iod-Komplex unter diesen Bedingungen so viel Iod freigesetzt hat, dass sämtliche Keime abgetötet wurden.

### Bezugszeichenliste:

1 = Zeolith-Iod-Eischlussverbindung (Granulat)
2 = Umverpackung (Umbeutel)
3 = festes Material
4 = poröses Material (Vlies etc.), Wundseite
5 = Klebeschicht (Hautkleber)
6 = Trägergewebe
7 = Wundkissen
8 = Deckpapier

## Patentansprüche

1. Zeolith-Iod-Einschlussverbindung zur Anwendung bei der Behandlung von Wunden bei Menschen und Tieren.

2. Zeolith-Iod-Einschlussverbindung gemäß Anspruch 1, **dadurch gekennzeichnet, dass** der Zeolith der Zeolith-Iod-Einschlussverbindung eine Porengröße von mindestens 5 Å aufweist.

3. Zeolith-Iod-Einschlussverbindung gemäß einem der Ansprüche 1 oder 2, **dadurch gekennzeichnet, dass** der Zeolith der Zeolith-Iod-Einschlussverbindung mit Iod im Bereich von 0, 1 Gew.-% bis zu seiner Maximalkapazität beladen ist.

4. Zeolith-Iod-Einschlussverbindung gemäß Anspruch 3, **dadurch gekennzeichnet, dass** der Zeolith der Zeolith-Iod-Einschlussverbindung mit Iod im Bereich von 1 bis 10 Gew.-% beladen ist.

5. Zeolith-Iod-Einschlussverbindung gemäß einem der Ansprüche 1 bis 4, **dadurch gekennzeichnet, dass** sich das Iod im wesentlichen in den Nanoporen des Zeolithen der Zeolith-Iod-Einschlussverbindung physikalisch gebunden befindet.

6. Zeolith-Iod-Einschlussverbindung gemäß einem der Ansprüche 1 bis 5, **dadurch gekennzeichnet, dass** die Zeolith-Iod-Einschlussverbindung granuliert, pelletiert oder gekörnt ist.

7. Zeolith-Iod-Einschlussverbindung gemäß Anspruch 6, **dadurch gekennzeichnet, dass** sie eine mittlere Korngröße von 0,5 bis 2 mm, insbesondere 1 mm aufweist.

8. Zeolith-Iod-Einschlussverbindung gemäß einem der Ansprüche 1 bis 5, **dadurch gekennzeichnet, dass** sie pulverförmig ist.

9. Verwendung einer Zeolith-Iod-Einschlussverbindung nach einem der Ansprüche 1 bis 8 zur Herstellung von Materialien zur Behandlung von Wunden, insbesondere Kompressen, Pflaster und/oder Verbandmaterial.

10. Verwendung nach Anspruch 9, **dadurch gekennzeichnet, dass** die Materialien weiterhin synthetische und/oder natürliche Polymere, insbesondere Polyacrylate, SIBS-Verbindungen, SEBS-Verbindungen, Naturkautschuke, Chitosane, Alginate, Hydrogele, Hydrokolloide und/oder Polyurethane enthalten

11. Verbandmaterial, Kompresse, Pflaster, Einwegwindeln, Damenbinden oder Stilleinlagen enthaltend die Zeolith-Iod-Einschlussverbindung nach einem der Ansprüche 1 bis 8.

12. Verbandmaterial, Kompresse, Pflaster, Einwegwindeln, Damenbinden oder Stilleinlage nach Anspruch 11**, dadurch gekennzeichnet, dass** sie synthetische und/oder natürliche Polymere, insbesondere Polyacrylate, SIBS-Verbindungen, SEBS-Verbindungen, Naturkautschuke, Chitosane, Alginate, Hydrogele, Hydrokolloide und/oder Polyurethane enthalten.

13. Pflaster enthaltend die Kompresse nach Anspruch **11.**

14. Materialien zur Anwendung bei der Behandlung von Wunden nach einem der Ansprüche 11 bis 13, **dadurch gekennzeichnet, dass** die Zeolith-Iod-Einschlussverbindung nach einem der Ansprüche 1 bis 8 wundseitig von einem porösen, textilen Faserstoff, insbesondere einem Vlies, Gewirk oder Gestrick sowie mit einer feuchtigkeitsdurchlässigen Folie abgedeckt ist.

## Claims

1. A zeolite-iodine inclusion compound for use in the treatment of wounds in humans and animals.

2. The zeolite-iodine inclusion compound according to claim 1, **characterized in that** the zeolite of said zeolite-iodine inclusion compound has a pore size of at least 5 Å.

3. The zeolite-iodine inclusion compound according to either of claims 1 or 2, **characterized in that** the zeolite of said zeolite-iodine inclusion compound is charged with iodine within a range of from 0.1% by weight up to its maximum capacity.

4. The zeolite-iodine inclusion compound according to claim 3, **characterized in that** the zeolite of said zeolite-iodine inclusion compound is charged with iodine within a range of from 1 to 10% by weight.

5. The zeolite-iodine inclusion compound according to any of claims 1 to 4, **characterized in that** the iodine is essentially physically bound in the nanopores of the zeolite of said zeolite-iodine inclusion compound.

6. The zeolite-iodine inclusion compound according to any of claims 1 to 5, **characterized in that** said zeolite-iodine inclusion compound is in the form of granules, pellets or grains.

7. The zeolite-iodine inclusion compound according to claim 6, **characterized by** having a mean grain size of from 0.5 to 2 mm, especially 1 mm.

8. The zeolite-iodine inclusion compound according to any of claims 1 to 5, **characterized by** being in powder form.

9. Use of a zeolite-iodine inclusion compound according to any of claims 1 to 8 for the preparation of materials for treating wounds, especially compresses, plasters and/or dressing materials.

10. The use according to claim 9, **characterized in that** said materials further contain synthetic and/or natural polymers, especially polyacrylates, SIBS compounds, SEBS compounds, natural rubbers, chitosans, alginates, hydrogels, hydrocolloids and/or polyurethanes.

11. Dressing material, compress, plasters, disposable diapers, sanitary towels or nursing pads containing the zeolite-iodine inclusion compound according to any of claims 1 to 8.

12. The dressing material, compress, patch, disposable diapers, sanitary towels or nursing pad according to claim 11, **characterized by** containing synthetic and/or natural polymers, especially polyacrylates, SIBS compounds, SEBS compounds, natural rubbers, chitosans, alginates, hydrogels, hydrocolloids and/or polyurethanes.

13. A plaster containing the compress according to claim 11.

14. Materials for use in the treatment of wounds according to any of claims 11 to 13, **characterized in that** the zeolite-iodine inclusion compound according to any of claims 1 to 8 is covered on the wound side by a porous textile fibrous material, especially a non-woven or knitted fabric, and by a moisture-permeable sheet.

## Revendications

1. Composé d'inclusion zéolite-iode pour l'application dans le traitement de blessures chez l'homme et les animaux.

2. Composé d'inclusion zéolite-iode selon la revendication 1, **caractérisé en ce que** la zéolithe dudit composé d'inclusion zéolite-iode a une taille de pores d'au moins 5 Å.

3. Composé d'inclusion zéolite-iode selon l'une des revendications 1 ou 2, **caractérisé en ce que** la zéolithe dudit composé d'inclusion zéolite-iode est chargé d'iode dans une gamme allant de 0,1 pour cent en poids jusqu'à sa capacité maximale.

4. Composé d'inclusion zéolite-iode selon la revendication 3, **caractérisé en ce que** la zéolithe dudit composé d'inclusion zéolite-iode est chargé d'iode dans une gamme allant de 1 à 10 pour cent en poids.

5. Composé d'inclusion zéolite-iode selon l'une quelconque des revendications 1 à 4, **caractérisé en ce que** l'iode est en substance physiquement lié dans les nanopores de la zéolithe dudit composé d'inclusion zéolite-iode.

6. Composé d'inclusion zéolite-iode selon l'une quelconque des revendications 1 à 5, **caractérisé en ce que** le composé d'inclusion zéolite-iode est sous forme de granules, pastilles ou graines.

7. Composé d'inclusion zéolite-iode selon la revendication 6, **caractérisé en ce qu'**il a une taille moyenne des particules allant de 0,5 à 2 mm, notamment 1 mm.

8. Composé d'inclusion zéolite-iode selon l'une quelconque des revendications 1 à 5, **caractérisé en ce qu'**il est sous forme de poudre.

9. Utilisation d'un composé d'inclusion zéolite-iode selon l'une quelconque des revendications 1 à 8 pour produire des matériels pour le traitement des blessures, notamment des compresses, des plâtres et/ou des matériels pour pansements.

10. Utilisation selon la revendication 9, **caractérisé en ce que** les matériels contiennent en outre des polymères synthétiques et/ou naturels, notamment des polyacrylates, des composés SIBS, des composés SEBS, des caoutchoucs naturels, des chitosanes, des alginates, des hydrogels, des hydrocolloïdes et/ou des polyuréthanes.

11. Matériel pour pansements, compresse, plâtre, couches à jeter, serviettes hygiéniques ou garnitures pour l'allaitement contenant le composé d'inclusion zéolite-iode selon l'une quelconque des revendications 1 à 8.

12. Matériel pour pansements, compresse, plâtres, couches à jeter, serviettes hygiéniques ou garnitures pour l'allaitement selon la revendication 11, **caractérisé en ce qu'**ils contiennent des polymères synthétiques et/ou naturels, notamment des polyacrylates, des composés SIBS, des composés SEBS, des caoutchoucs naturels, des chitosanes, des alginates, des hydrogels, des hydrocolloïdes et/ou des polyuréthanes.

13. Plâtres contenant la compresse selon la revendication 11.

14. Matériels pour l'application dans le traitement de blessures selon l'une quelconque des revendications 11 à 13, **caractérisé en ce que** le composé d'inclusion zéolite-iode selon l'une quelconque des revendications 1 à 8 est couvert, au coté de la blessure, d'un matériau fibreux textile poreux, notamment un tissu non-tissé, tissu à mailles ou tricot, et d'une feuille perméable à l'humidité.
